# EUROPEAN PATENT APPLICATION

(11) **EP 2 018 865 A2**
(43) Date of publication of application: **28.01.2009**
(21) Application number: 08166467.4
(22) Date of filing: 02.09.2004
(51) Int. Cl.: A61K 31/497, A61K 31/663, A61K 38/09, A61P 35/00

(54) **COMBINATION COMPRISING N-(3-METHOXY-5-METHYLPYRAZIN-2-YL)-2-(4-[1,3,4-OXADIAZOL-2-YL]PHENYL)PYRIDINE-3-SULPHONAMIDE AND AN LHRH ANALOGUE AND/OR A BISPHOSPHONATE**

(30) Priority: 05.09.2003 GB 0320806
(62) Divisional of application: 04768282.8
(71) Applicant: AstraZeneca AB, 151 85 Södertälje (SE)
(72) Inventor: Gallagher, Neil, Macclesfield, Cheshire SK10 4TG (GB); Curwen, Jon, Owen, Macclesfield, Cheshire SK10 4TG (GB)

(57) **Abstract**

A combination, comprising N-(3-methoxy-5-methylpyrazin-2-yl)-2-(4-[1,3,4-oxadiazol-2-yl]phenyl)pyridine-3-sulphonamide, or a pharmaceutically acceptable salt thereof, and an LHRH analogue is described.

## Description

The present invention relates to a combination comprising *N*-(3-methoxy-5-methylpyrazin-2-yl)-2-(4-[1,3,4-oxadiazol-2-yl]phenyl)pyridine-3-sulphonamide, or a pharmaceutically acceptable salt thereof, hereafter "Compound **(I)**", and a luteinizing hormone releasing hormone analogue (hereafter LHRH analogue). The present invention further relates to a combination comprising Compound **(I)** and a diphosphonic acid derivative (hereafter bisphosphonate) and a combination comprising Compound **(I),** an LHRH analogue and a bisphosphonate.

These combinations are useful for the treatment or prophylaxis of cancer. The invention also relates to a pharmaceutical composition comprising such combinations and to the use thereof in the manufacture of a medicament for use in the treatment or prophylaxis of cancer, in particular prostate cancer.

Cancer affects an estimated 10 million people worldwide. This figure includes incidence, prevalence and mortality. More than 4.4 million cancer cases are reported from Asia, including 2.5 million cases from Eastern Asia, which has the highest rate of incidence in the world. By comparison, Europe has 2.8 million cases, North America 1.4 million cases, and Africa 627,000 cases.

In the UK and US, for example, more than one in three people will develop cancer at some point in their life. Cancer mortality in the U.S. is estimated to account for about 600,000 a year, about one in every four deaths, second only to heart disease in percent of all deaths, and second to accidents as a cause of death of children 1-14 years of age. The estimated cancer incidence in the U.S. is now about 1,380,000 new cases annually, exclusive of about 900,000 cases of non-melanotic (basal and squamous cell) skin cancer.

Cancer is also a major cause of morbidity in the UK with nearly 260,000 new cases (excluding non-melanoma skin cancer) registered in 1997. Cancer is a disease that affects mainly older people, with 65% of cases occurring in those over 65. Since the average life expectancy in the UK has almost doubled since the mid nineteenth century, the population at risk of cancer has grown. Death rates from other causes of death, such as heart disease, have fallen in recent years while deaths from cancer have remained relatively stable. The result is that 1 in 3 people will be diagnosed with cancer during their lifetime and 1 in 4 people will die from cancer. In people under the age of 75, deaths from cancer outnumber deaths from diseases of the circulatory system, including ischaemic heart disease and stroke. In 2000, there were 151,200 deaths from cancer. Over one fifth (22 per cent) of these were from lung cancer, and a quarter (26 per cent) from cancers of the large bowel, breast and prostate.

Worldwide, the incidence and mortality rates of certain types of cancer (of stomach, breast, prostate, skin, and so on) have wide geographical differences which are attributed to racial, cultural, and especially environmental influences. There are over 200 different types of cancer but the four major types, lung, breast, prostate and colorectal, account for over half of all cases diagnosed in the UK and US. Prostate cancer is the fourth most common malignancy among men worldwide, with an estimated 400,000 new cases diagnosed annually, accounting for 3.9 percent of all new cancer cases.

Current options for treating cancers include surgical resection, external beam radiation therapy and / or systemic chemotherapy. These are partially successful in some forms of cancer, but are not successful in others. There is a clear need for new therapeutic treatments.

Recently, endothelin A receptor antagonists have been identified as potentially of value in the treatment of cancer (Cancer Research, 56, 663-668, February 15th, 1996 and Nature Medicine, Volume 1, Number 9, September 1999, 944-949).

The endothelins are a family of endogenous 21 amino acid peptides comprising three isoforms, endothelin-1, endothelin-2 and endothelin-3. The endothelins are formed by cleavage of the Trp²¹-Val²² bond of their corresponding proendothelins by an endothelin converting enzyme. The endothelins are among the most potent vasoconstrictors known. They exhibit a wide range of other activities including stimulation of cell proliferation and mitogenesis, inhibition of apoptosis, extravasation and chemotaxis, and also interact with a number of other vasoactive agents.

The endothelins are released from a range of tissue and cell sources including vascular endothelium, vascular smooth muscle, kidney, liver, uterus, airways, intestine and leukocytes. Release can be stimulated by hypoxia, shear stress, physical injury and a wide range of hormones and cytokines. Elevated endothelin levels have been found in a number of disease states in man including cancers.

LHRH is a decapeptide that is secreted by the hypothalamus into the hypophyseal portal circulation in response to neural and/or chemical stimuli, causing the biosynthesis and release of luteinizing hormone (LH) and follicle-stimulating hormone (FSH) by the pituitary. LHRH is also known by other names, Gonadotropin releasing hormone (GnRH), gonadoliberin, FSH releasing hormone (FSH RH) and LH/FSH releasing factor (LH/FSH RF).

The role of gonadal androgens in propagating and maintaining the growth of prostate cancer has long been recognised (Huggins C, Hodges CV. Cancer Res 1941;1:293). LHRH plays an important role in regulating the action of LH and FSH (by regulation of their levels), and thus has a role in regulating the levels of gonadal steroids in both sexes, including the sex hormones progesterone, oestrogens and androgens. More discussion of LHRH can be found in WO 97/14697, the disclosure of which is incorporated herein by reference.

Medical and surgical castration are commonly used for the treatment of prostate cancer and LHRH analogues are frequently used to induce medical castration for the management of patients with early and advanced prostate cancer. They have proven effective in the treatment of certain conditions which require inhibition of LH/FSH release. In particular, LHRH-based therapies have proven effective in the treatment of endometriosis, uterine fibroids, polycystic ovarian disease, precocious puberty and several gonadal steroid -dependent neoplasia, most notably cancers of the prostate, breast and ovary. LHRH analogues have also been utilized in various assisted fertilization techniques and have been investigated as a potential contraceptive in both men and women. They have also shown possible utility in the treatment of pituitary gonadotrophe adenomas, sleep disorders such as sleep apnea, irritable bowel syndrome, premenstrual syndrome, benign prostatic hyperplasia, hirsutism, as an adjunct to growth hormone therapy in growth hormone deficient children, and in murine models of lupus.

Bisphosphonates are diphosphonic acid derivatives that are capable of regulating metal cations content (especially calcium content) in humans. In particular, they have a pronounced regulatory action on the calcium metabolism of warm-blooded animals. They are thus useful in the treatment of diseases connected with content and circulation of these cations particularly the retardation of bone decalcification. Most particularly, they effect a marked inhibition of bone resorption in rats, as can be demonstrated in the experimental procedure described in Acta Endrocinol. 78, 613-24 (1975).

The clinical use of bisphosphonates has increased dramatically in the past decade. The most common indication for these compounds is osteoporosis, but their use in osteolytic bone disease has increased rapidly. The FDA approved pamidronate in 1995 for the treatment of normocalcemic patients with myeloma. In 1996 they issued a new approval for patients with osteolytic lesions of metastatic breast cancer prompting the widespread use of pamidronate in patients with tumors that metastasize to bone. Although the effect of bisphosphonates on osteoclast-mediated bone resorption has been known and well documented for many years, the exact mechanism of that inhibition is still not completely defined.

The present inventors have unexpectedly found that:
i) the combination use of Compound **(I)** and LHRH analogues; or
ii) the combination use of Compound **(I),** and bisphosphonates; or
iii) the combination use of Compound **(I),** LHRH analogues and bisphosphonates; can have a particular benefit in the treatment of cancer.

Therefore according to the present invention, there is provided a combination, comprising Compound **(I),** and an LHRH analogue.

According to a further aspect of the present invention, there is provided a combination, comprising Compound **(I),** and a bisphosphonate.

According to a further aspect of the present invention, there is provided a combination, comprising Compound **(I),** an LHRH analogue and a bisphosphonate.

Herein where the term "LHRH analogue" is used it is to be understood that this refers to any chemical compound, or a pharmaceutically acceptable salt thereof, including small molecules and peptides, which acts as an agonist or antagonist at the LHRH receptor, whether by an interaction with the LHRH binding site or by an allosteric mechanism, i.e. acts at a position on the LHRH receptor different to the LHRH binding site. In one aspect of the invention an "LHRH analogue" refers to an LHRH antagonist or a pharmaceutically acceptable salt thereof. In one aspect of the invention an "LHRH analogue" refers to an LHRH agonist or a pharmaceutically acceptable salt thereof. In a further aspect of the invention an "LHRH analogue" refers to a combination of an LHRH antagonist or a pharmaceutically acceptable salt thereof and an LHRH agonist or a pharmaceutically acceptable salt thereof.

A "bisphosphonate" is a compound, or a pharmaceutically acceptable salt thereof, capable of regulating metal cations content (especially calcium content) in humans and is a compound containing two carbon phosphorous bonds. For further explanation of the term "bisphosphonate" the readers attention is drawn to Endocrine Reviews, 1998, 19(1): 80-100, the content of which is incorporated herein by reference.

Herein, where the term "combination" is used it is to be understood that this refers to simultaneous, separate or sequential administration. In one aspect of the invention "combination" refers to simultaneous administration. In another aspect of the invention "combination" refers to separate administration. In a further aspect of the invention "combination" refers to sequential administration. Where the administration is sequential or separate, the delay in administering the second component should not be such as to lose the benefit of the synergistic effect of the combination.

In one aspect, where a compound or a pharmaceutically acceptable salt thereof, is referred to this refers to the compound only. In another aspect this refers to a pharmaceutically acceptable salt of the compound.

Where cancer is referred to, particularly it refers to oesophageal cancer, myeloma, hepatocellular, pancreatic, cervical cancer, ewings tumour, neuroblastoma, kaposis sarcoma, ovarian cancer, breast cancer, colorectal cancer, prostate cancer, bladder cancer, melanoma, lung cancer - non small cell lung cancer (NSCLC), and small cell lung cancer (SCLC), gastric cancer, head and neck cancer, brain cancer, renal cancer, lymphoma and leukaemia. More particularly it refers to prostate cancer. In addition, more particularly it refers to SCLC, NSCLC, colorectal cancer, ovarian cancer and / or breast cancer. In addition, more particularly it refers to SCLC. In addition, more particularly it refers to NSCLC. In addition, more particularly it refers to colorectal cancer. In addition, more particularly it refers to ovarian cancer. In addition, more particularly it refers to breast cancer. Furthermore, more particularly it refers to bladder cancer, oesophageal cancer, gastric cancer, melanoma, cervical cancer and / or renal cancer. In addition it refers to endometrial, liver, stomach, thyroid, rectal and / or brain cancer. In another aspect of the invention, the cancer is not melanoma.In another embodiment of the invention, particularly the cancer is in a metastatic state, and more particularly the cancer produces metastases to the bone. In a further embodiment of the invention, particularly the cancer is in a metastatic state, and more particularly the cancer produces skin metastases. In a further embodiment of the invention, particularly the cancer is in a metastatic state, and more particularly the cancer produces lymphatic metastases. In a further embodiment of the invention, the cancer is in a non-metastatic state.

Where the treatment of cancer is referred to particularly this is the treatment of cancerous tumours expressing endothelin A. This treatment is in terms of one or more of the extent of the response, the response rate, the time to disease progression and the survival rate. It is further expected that the combination use of Compound **(I)** and particular LHRH analogues will have a beneficial effect in preventing the onset of cancer in warm-blooded animals, such as man.

Particular compounds, or pharmaceutically acceptable salts thereof possessing LHRH analogue activity include Azaline B, A-198401, A-75998, A-76154, A-84861, abarelix, AN-152, AN-207, Antide, avorelin, cetrorelix, D-21775, D-23487, D-26344, D-63153, D-85108, degarelix, deslorelin, detirelix, FE 200486, ganirelix, gonadimmune, goserelin, histrelin, leuprolide, leuprorelin, metarelin, nafarelin, NBI-42902 (Neurocrine), Org-30850, PH-45 (Pherin Corp), PTL-03001, ramorelix, RWJ-47428-021, SPD-424, surfagon, T-66 (Matrix Therapeutics Ltd), TAK-013, TAK-810, teverelix, triptorelin acetate, triptorelin pamoate, vomeropherin or ZK-157348.

Particular LHRH analogues are peptides or peptide derivatives.

Examples of particular LHRH agonists include, but are not limited to;
i) buserelin (US Patent 4 024 248)
   (pyr)Glu-His-Trp-Ser-Tyr-D-Ser(Bu^{t})⁶-Leu-Arg-Pro-NHCH₂CH₃
ii) triptorelin (US Patent 4 10 125)
   (pyr)Glu-His-Trp-Ser-Tyr-Trp-Leu-Arg-Pro-Gly-NH2
iii) leuprorelin (Us Patent 4 005 063)
   (pyr)Glu-His-Trp-Ser-Tyr-D-Leu-Leu-Arg-Pro-NHCH2CH3
iv) goserelin (US Patent 4 100 274)
   (pyr)Glu-His-Trp-Ser-Tyr- D-Ser(Bu^{t})⁶-Leu-Arg-Pro-(Azygly)NH₂
v) deslorelin (US Patent 4 659 695)
   (pyr)Glu-His-Trp-Ser-Tyr-D-Trp-Leu-Arg-Pro-NH-CH₂-CH₂-NH₂
vi) histerelin (US Patent 4 244 946)
   (pyr)Glu-His-Trp- Ser-Tyr-D-His(Bzl)-Leu-Arg-Pro-NH-CH2-CH3
vii) avorelin (US 5 668 254)
   (pyr)Glu-His-Trp-Ser-Tyr-D-Trp(2-Me)-Leu-Arg-Pro-NH-CH2-CH3
viii) nafarelin (US Patent 4 234 571)
   (pyr)Glu-His-Trp-Ser-Tyr-D-Nal(2)-Leu-Arg-Pro-NH-CH₂-CH₃;
lutrelin, cystorelin, gonadorelin or detirelix.

Particularly the LHRH agonist is selected from leuprorelin, buserelin, triptorelin and goserelin. More particularly the LHRH agonist is goserelin.

Examples of suitable LHRH antagonists include, but are not limited to, antide, abarelix, antarelix, cetrorelix, azaline, ganirelix and those disclosed in US Patents 5 470 947 (Folkers); 5 413 990 and 5 300 492 (Haviv); 5 371 070 (Koerber); 5 296 468 (Hoeger); 5 171 635 (Janaky); 5 003 011 and 4 431 635 (Coy); 4 992 421 (De); 4 801 577 (Nestor); and 4 851 385, 4 689 396 and 5 843 901 (Roeske).

Further examples of suitable LHRH antagonists include, but are not limited to the compounds described in WO 02/066477, WO 02066478, WO 02/066459, WO 02/092565, PCT/GB03/003603 and PCT/GB03/003606, and the compounds described in these applications, particularly the compounds of claim 1 and the named examples are incorporated herein by reference.

Particular bisphosphonates for use in the present invention are selected from tiludronic acid, ibandronic acid, incadronic acid, risedronic acid, zoledronic acid, clodronic acid, neridronic acid, pamidronic acid, alendronic acid, minodronic acid, olpadronic acid, TRK 530, CGP 47072, calcium clodronate or EB 1053. Further particular bisphosphonates for use in the present invention are selected from etidronic acid, PNU-91638, NE-21650, NE-58025, NE-10790 or NE-10446.

Particular combinations of the present invention include:
- Compound **(I)** and leuprorelin, or a pharmaceutically acceptable salt thereof;
- Compound **(I)** and goserelin, or a pharmaceutically acceptable salt thereof;
- Compound **(I)** and abarelix, or a pharmaceutically acceptable salt thereof;
- Compound **(I)** and cetrorelix, or a pharmaceutically acceptable salt thereof;
- Compound **(I)** and risedronic acid, or a pharmaceutically acceptable salt thereof;
- Compound **(I)** and zoledronic acid, or a pharmaceutically acceptable salt thereof;
- Compound **(I)** and clodronic acid, or a pharmaceutically acceptable salt thereof;
   and
- Compound **(I)** and pamidronic acid, or a pharmaceutically acceptable salt thereof.
- Compound **(I)** and alendronic acid, or a pharmaceutically acceptable salt thereof.

Suitable pharmaceutically-acceptable salts include, for example, salts with alkali metal (such as sodium, potassium or lithium), alkaline earth metals (such as calcium or magnesium), ammonium salts, and salts with organic bases affording physiologically acceptable cations, such as salts with methylamine, dimethylamine, trimethylamine, piperidine and morpholine. In addition, for those compounds which are sufficiently basic, suitable pharmaceutically-acceptable salts include, pharmaceutically-acceptable acid-addition salts with hydrogen halides, sulphuric acid, phosphoric acid and with organic acids such as citric acid, maleic acid, methanesulphonic acid and p-toluenesulphonic acid. Alternatively, the compounds may exist in zwitterionic form.

Therefore according to the present invention, there is provided a combination, comprising Compound **(I)** and an LHRH analogue for use as a medicament.

Therefore according to the present invention, there is provided a combination, comprising Compound **(I)** and a bisphosphonate for use as a medicament.

Therefore according to the present invention, there is provided a combination, comprising Compound **(I),** an LHRH analogue and a bisphosphonate for use as a medicament.

According to a further aspect of the invention there is provided a pharmaceutical composition which comprises Compound **(I)** and an LHRH analogue in association with a pharmaceutically acceptable diluent or carrier.

According to a further aspect of the invention there is provided a pharmaceutical composition which comprises Compound **(I)** and a bisphosphonate in association with a pharmaceutically acceptable diluent or carrier.

According to a further aspect of the invention there is provided a pharmaceutical composition which comprises Compound **(I),** an LHRH analogue and a bisphosphonate in association with a pharmaceutically acceptable diluent or carrier.

According to a further aspect of the invention there is provided a pharmaceutical composition which comprises Compound **(I),** in association with a pharmaceutically acceptable diluent or carrier, in combination with a pharmaceutical composition which comprises an LHRH analogue in association with a pharmaceutically acceptable diluent or carrier.

According to a further aspect of the invention there is provided a pharmaceutical composition which comprises Compound **(I),** in association with a pharmaceutically acceptable diluent or carrier, in combination with a pharmaceutical composition which comprises a bisphosphonate in association with a pharmaceutically acceptable diluent or carrier.

According to a further aspect of the invention there is provided a pharmaceutical composition which comprises Compound **(I),** in association with a pharmaceutically acceptable diluent or carrier, in combination with a pharmaceutical composition which comprises an LHRH analogue in association with a pharmaceutically acceptable diluent or carrier and a bisphosphonate in association with a pharmaceutically acceptable diluent or carrier.

Therefore according to the present invention, there is provided a method of treating cancer, in a warm-blooded animal, such as man, in need of such treatment which comprises administering to said animal an effective amount of Compound **(I)** in combination with an effective amount of an LHRH analogue.

Therefore according to the present invention, there is provided a method of treating cancer, in a warm-blooded animal, such as man, in need of such treatment which comprises administering to said animal an effective amount of Compound **(I)** in combination with an effective amount of a bisphosphonate.

Therefore according to the present invention, there is provided a method of treating cancer, in a warm-blooded animal, such as man, in need of such treatment which comprises administering to said animal an effective amount of Compound **(I)** in combination with an effective amount of an LHRH analogue and an effective amount of a bisphosphonate.

For the avoidance of doubt, where the treatment of cancer is indicated, it is to be understood that this also refers to the prevention of metastases and the treatment of metastases, i.e. cancer spread. Therefore the combination of the present invention could be used to treat a patient who has no metastases to stop them occurring, or to lengthen the time period before they occur, and to a patient who already has metastases to treat the metastases themselves. Furthermore the treatment of cancer also refers to treatment of an established primary tumour or tumours and developing primary tumour or tumours. In one aspect of the invention the treatment of cancer relates to the prevention of metastases. In another aspect of the invention the treatment of cancer relates to the treatment of metastases. In another aspect of the invention the treatment of cancer relates to treatment of an established primary tumour or tumours or developing primary tumour or tumours. Herein, the treatment of cancer also refers to the prevention of cancer *per se*.

In addition the treatment of cancer also refers to the production of an anti-angiogenic effect in a warm blooded animal.

According to a further aspect of the present invention there is provided a kit comprising Compound **(I)** and an LHRH analogue; optionally with instructions for use.

According to a further aspect of the present invention there is provided a kit comprising Compound **(I),** and a bisphosphonate; optionally with instructions for use.

According to a further aspect of the present invention there is provided a kit comprising Compound **(I),** an LHRH analogue and a bisphosphonate; optionally with instructions for use.

According to a further aspect of the present invention there is provided a kit comprising:
a) Compound **(I),** in a first unit dosage form;
b) an LHRH analogue; in a second unit dosage form; and
c) container means for containing said first and second dosage forms; and optionally
d) with instructions for use.

An example of a unit dosage from for Compound **(I)** might be a tablet for oral formulation, see that described herein below. An example of a unit dosage from for an LHRH analogue might be a prolonged release formulation for an LHRH agonist (see herein below) or a prolonged release formulation or a tablet formulation for an LHRH antagonist (see herein below).

According to a further aspect of the present invention there is provided a kit comprising:
a) Compound **(I),** in a first unit dosage form;
b) a bisphosphonate; in a second unit dosage form; and
c) container means for containing said first and second dosage forms; and optionally
d) with instructions for use.

An example of a unit dosage from for a bisphosphonate might be a tablet for oral formulation, see herein below.

According to a further aspect of the present invention there is provided a kit comprising:
a) Compound **(I),** in a first unit dosage form;
b) an LHRH analogue; in a second unit dosage form; and
c) a bisphosphonate; in a third unit dosage form; and
d) container means for containing said first, second and third dosage forms; and optionally
e) with instructions for use.

According to a further aspect of the present invention there is provided a kit comprising:
a) Compound **(I),** together with a pharmaceutically acceptable diluent or carrier, in a first unit dosage form;
b) an LHRH analogue, in a second unit dosage form; and
c) container means for containing said first and second dosage forms; and optionally
d) with instructions for use.

According to a further aspect of the present invention there is provided a kit comprising:
a) Compound **(I),** together with a pharmaceutically acceptable diluent or carrier, in a first unit dosage form;
b) a bisphosphonate, in a second unit dosage form; and
c) container means for containing said first and second dosage forms; and optionally
d) with instructions for use.

According to a further aspect of the present invention there is provided a kit comprising:
a) Compound **(I),** together with a pharmaceutically acceptable diluent or carrier, in a first unit dosage form;
b) an LHRH analogue, in a second unit dosage form; and
c) a bisphosphonate, in a third unit dosage form; and
d) container means for containing said first, second and third dosage forms; and optionally
e) with instructions for use.

According to a further aspect of the invention there is provided a pharmaceutical composition which comprises Compound **(I)** and an LHRH analogue in association with a pharmaceutically acceptable diluent or carrier for use in the treatment of cancer.

According to a further aspect of the invention there is provided a pharmaceutical composition which comprises Compound **(I)** and a bisphosphonate in association with a pharmaceutically acceptable diluent or carrier for use in the treatment of cancer.

According to a further aspect of the invention there is provided a pharmaceutical composition which comprises Compound **(I),** an LHRH analogue in association with a pharmaceutically acceptable diluent or carrier and a bisphosphonate in association with a pharmaceutically acceptable diluent or carrier for use in the treatment of cancer.

According to a further aspect of the invention there is provided a pharmaceutical composition which comprises Compound **(I),** in association with a pharmaceutically acceptable diluent or carrier, in combination with a pharmaceutical composition which comprises an LHRH analogue in association with a pharmaceutically acceptable diluent or carrier for use in the treatment of cancer.

According to a further aspect of the invention there is provided a pharmaceutical composition which comprises Compound **(I),** in association with a pharmaceutically acceptable diluent or carrier, in combination with a pharmaceutical composition which comprises a bisphosphonate in association with a pharmaceutically acceptable diluent or carrier for use in the treatment of cancer.

According to a further aspect of the invention there is provided a pharmaceutical composition which comprises Compound **(I),** in association with a pharmaceutically acceptable diluent or carrier, in combination with a pharmaceutical composition which comprises an LHRH analogue in association with a pharmaceutically acceptable diluent or carrier and a bisphosphonate in association with a pharmaceutically acceptable diluent or carrier for use in the treatment of cancer.

The pharmaceutical compositions may be in a form suitable for oral administration, for example as a tablet or capsule, for parenteral injection (including intravenous, subcutaneous, intramuscular, intravascular or infusion) as a sterile solution, suspension or emulsion, for topical administration as an ointment or cream or for rectal administration as a suppository. In general the above compositions may be prepared in a conventional manner using conventional excipients.

For example Compound **(I)** can be formulated as a tablet using the following excipients:
Compound **(I);**
Lactose monohydrate (filler);
Croscarmellose sodium (disintegrant);
Povidone (binder);
Magnesium stearate (lubricant);
Hypromellose (film coat component);
Polyethylene glycol 300 (film coat component); and
Titanium dioxide (film coat component).

According to a further aspect of the present invention there is provided a kit comprising Compound **(I)** and an LHRH analogue; optionally with instructions for use; for use in the treatment of cancer.

According to a further aspect of the present invention there is provided a kit comprising Compound **(I),** and a bisphosphonate; optionally with instructions for use; for use in the treatment of cancer.

According to a further aspect of the present invention there is provided a kit comprising Compound **(I),** an LHRH analogue and a bisphosphonate; optionally with instructions for use; for use in the treatment of cancer.

According to a further aspect of the present invention there is provided a kit comprising:
a) Compound **(I),** in a first unit dosage form;
b) an LHRH analogue in a second unit dosage form; and
c) container means for containing said first and second dosage forms; and optionally
d) with instructions for use;
for use in the treatment of cancer.

According to a further aspect of the present invention there is provided a kit comprising:
a) Compound **(I),** in a first unit dosage form;
b) a bisphosphonate in a second unit dosage form; and
c) container means for containing said first and second dosage forms; and optionally
d) with instructions for use;
for use in the treatment of cancer.

According to a further aspect of the present invention there is provided a kit comprising:
a) Compound **(I),** in a first unit dosage form;
b) an LHRH analogue in a second unit dosage form; and
c) a bisphosphonate in a third unit dosage form; and
d) container means for containing said first, second and third dosage forms; and optionally
e) with instructions for use;
for use in the treatment of cancer.

According to a further aspect of the present invention there is provided a kit comprising:
a) Compound **(I),** together with a pharmaceutically acceptable diluent or carrier, in a first unit dosage form;
b) an LHRH analogue in a second unit dosage form; and
c) container means for containing said first and second dosage forms; and optionally
d) with instructions for use;
for use in the treatment of cancer.

According to a further aspect of the present invention there is provided a kit comprising:
a) Compound **(I),** together with a pharmaceutically acceptable diluent or carrier, in a first unit dosage form;
b) a bisphosphonate in a second unit dosage form; and
c) container means for containing said first and second dosage forms; and optionally
d) with instructions for use;
for use in the treatment of cancer.

According to a further aspect of the present invention there is provided a kit comprising:
a) Compound **(I),** together with a pharmaceutically acceptable diluent or carrier, in a first unit dosage form;
b) an LHRH analogue in a second unit dosage form; and
c) a bisphosphonate in a second unit dosage form; and
d) container means for containing said first, second and third dosage forms; and optionally
e) with instructions for use;
for use in the treatment of cancer.

According to another feature of the invention there is provided the use of Compound **(I),** in combination with an LHRH analogue in the manufacture of a medicament for use in the treatment of cancer, in a warm-blooded animal, such as man.

According to another feature of the invention there is provided the use of Compound **(I),** in combination with a bisphosphonate in the manufacture of a medicament for use in the treatment of cancer, in a warm-blooded animal, such as man.

According to another feature of the invention there is provided the use of Compound **(I),** in combination with an LHRH analogue and a bisphosphonate in the manufacture of a medicament for use in the treatment of cancer, in a warm-blooded animal, such as man.

According to another feature of the invention there is provided the use of Compound **(I),** in combination with an LHRH analogue in the treatment of cancer, in a warm-blooded animal, such as man.

According to another feature of the invention there is provided the use of Compound **(I),** in combination with a bisphosphonate in the treatment of cancer, in a warm-blooded animal, such as man.

According to another feature of the invention there is provided the use of Compound **(I),** in combination with an LHRH analogue and a bisphosphonate in the treatment of cancer, in a warm-blooded animal, such as man.

According to a further aspect of the present invention there is provided a combination comprising Compound **(I)** and an LHRH analogue for use in the treatment of cancer.

According to a further aspect of the present invention there is provided a combination comprising Compound **(I),** and a bisphosphonate for use in the treatment of cancer.

According to a further aspect of the present invention there is provided a combination comprising Compound **(I),** an LHRH analogue and a bisphosphonate for use in the treatment of cancer.

According to a further aspect of the present invention there is provided a combination treatment comprising the administration of an effective amount of Compound **(I),** optionally together with a pharmaceutically acceptable diluent or carrier, in combination with an effective amount of an LHRH analogue optionally together with a pharmaceutically acceptable diluent or carrier to a warm-blooded animal, such as man in need of such therapeutic treatment for use in the treatment of cancer.

According to a further aspect of the present invention there is provided a combination treatment comprising the administration of an effective amount of Compound **(I),** optionally together with a pharmaceutically acceptable diluent or carrier, in combination with an effective amount of a bisphosphonate optionally together with a pharmaceutically acceptable diluent or carrier to a warm-blooded animal, such as man in need of such therapeutic treatment for use in the treatment of cancer.

According to a further aspect of the present invention there is provided a combination treatment comprising the administration of an effective amount of Compound **(I),** optionally together with a pharmaceutically acceptable diluent or carrier, in combination with an effective amount of an LHRH analogue optionally together with a pharmaceutically acceptable diluent or carrier and an effective amount of a bisphosphonate optionally together with a pharmaceutically acceptable diluent or carrier to a warm-blooded animal, such as man in need of such therapeutic treatment for use in the treatment of cancer.

The amount of Compound **(I),** or a pharmaceutically acceptable salt thereof, administered would be that sufficient to provide the desired pharmaceutical effect. For instance, Compound **(I)** could be administered to a warm-blooded animal orally, at a unit dose less than 1g daily but more than 2.5mg. Particularly Compound **(I)** could be administered to a warm-blooded animal, at a unit dose of less than 250 mg per day. In another aspect of the invention, Compound **(I)** could be administered to a warm-blooded animal, at a unit dose of less than 130 mg per day. In a further aspect of the invention, Compound **(I)** could be administered to a warm-blooded animal, at a unit dose of less than 50 mg per day.

LHRH analogues, particularly LHRH agonists would normally be administered as a prolonged release formulation in order to enhance the therapeutic effectiveness of the drug. A number of prolonged release formulations containing LHRH analogues are known and can be achieved by administering the drug in the form of a biodegradable polymer matrix which releases the drug over a prolonged period. Suitable biodegradable polymers are polylactides. The term 'polylactide' is used in a generic sense to include polymers of lactic acid alone, copolymers of lactic and glycolic acid, mixtures of such polymers, mixtures of such copolymers and mixtures of such polymers and copolymers, the lactic acid being either in racemic or optically active form.

One approach has been to develop microparticle or microcapsule formulations comprising the biodegradable polymer, typically a poly(lactide-co-glycolide) co-polymer, in which the LHRH analogue is microencapsulated.

EP 839 525 describes the preparation of microcapsules containing LHRH analogues using an emulsion based process wherein a water-in-oil emulsion is prepared comprising an inner aqueous phase containing the LHRH analogue and an outer oil phase comprising a solution of a polymer of lactic acid in an organic solvent. Generally, microencapsulated LHRH analogues are suitable for delivering the LHRH analogue for up to 3 months.

EP 058 481 describes monolithic implants comprising a biodegradable poly(lactide-co-glycolide) co-polymer and an LHRH agonist.

WO 03/022297 describes monolithic implants prepared using a combination of a specific polylactide polymer and a LHRH analogue which continuously releases the LHRH analogue over a period of at least six months when placed in an aqueous physiological-type environment.

WO 03/022243 describes the sustained release of microcrystalline peptide suspensions containing LHRH agonists.

As an alternative Eligard (an LHRH agonist) is sold as a flowable polymeric formulation composed of poly (DL-lactide) which is suspended in a non-aqueous solvent N-methyl-2-pyrrolidone.

An LHRH antagonist as well as being delivered by any of the prolonged release methods listed above could also be prepared for oral administration using standard techniques and known excipients.

The LHRH analogue will normally be administered to a warm-blooded animal at a unit dose, for example, from about 3-4mg per month, or 10-11mg every 3 months, of active ingredient. When the LHRH analogue is goserelin, a sustained release subcutaneous formulation containing 3.6mg per month, or 10.6mg every 3 months, of active ingredient Conveniently the daily oral dose of leuprorelin is 3.75mg per month, or 11.25mg every 3 months. The optimum dosage however, may be determined by the practitioner who is treating any particular patient.

The bisphosphonate will normally be administered to a warm-blooded animal at a unit dose, of an amount known to the skilled practitioner as a therapeutically effective dose. For a single dosage form, the active ingredients may be compounded with an appropriate and convenient amount of excipients which may vary from about 5 to about 98 percent by weight of the total composition. Dosage unit forms will generally contain about 20 mg to about 500 mg of each active ingredient. However the daily dose will necessarily be varied depending upon the host treated, the particular route of administration, and the severity of the illness being treated. Accordingly the optimum dosage may be determined by the practitioner who is treating any particular patient.

The dosage of each of the drugs and their proportions have to be composed so that the best possible treatment effects, as defined by national and international guidelines (which are periodically reviewed and re-defined), will be met.

## Claims

1. A combination, comprising N-(3-methoxy-5-methylpyrazin-2-yl)-2-(4-[1,3,4-oxadiazol-2-yl]phenyl)pyridine-3-sulphonamide, or a pharmaceutically acceptable salt thereof, and an LHRH analogue.

2. A combination according to claim 1 wherein the LHRH analogue is an LHRH agonist.

3. A combination according to claim 2 wherein the LHRH agonist is selected from leuprorelin, buserelin, triptorelin and goserelin.

4. A combination according to claims 2 or 3 wherein the LHRH agonist is goserelin.

5. A combination according to claim 1 wherein the LHRH analogue is an LHRH antagonist.

6. A combination according to claim 5 wherein the LHRH antagonist is selected from antide, abarelix, antarelix, cetrorelix, azaline or ganirelix.

7. A combination as claimed in any one of claims 1-6 for use as a medicament.

8. A combination as claimed in any one of claims 1-6 for use in the treatment of a cancer.

9. A combination as claimed in any one of claims 1-6 for use in the treatment of prostate cancer.

10. A pharmaceutical composition as claimed in any one of claims 1-6 in association with a pharmaceutically acceptable diluent or carrier.

11. A pharmaceutical composition which comprises a combination as claimed in any one of claims 1-6 in association with a pharmaceutically acceptable diluent or carrier for use in the treatment of cancer.

12. The use of a combination as claimed in any one of claims 1-6 in the manufacture of a medicament for use in the treatment of cancer, in a warm-blooded animal, such as man.

13. The use according to claim 12 wherein the cancer is prostate cancer.
